# EUROPEAN PATENT SPECIFICATION

(11) **EP 0 405 517 B1**
(45) Date of publication and mention of the grant of the patent: **26.10.1994**
(21) Application number: 90112282.0
(22) Date of filing: 27.06.1990
(51) Int. Cl.: C07C 53/48, C07C 51/58

(54) **Process for preparing bromodifluoroacetylfluoride**
Verfahren zur Herstellung von Bromdifluoracetylfluorid
Procédé pour préparer un fluorure de difluorobromoacétyle

(30) Priority: 28.06.1989 IT 2101689
(43) Date of publication of application: 02.01.1991
(73) Proprietor: AUSIMONT S.p.A., I-20121 Milano (IT)
(72) Inventor: Modena, Silvana, Dr., I-20052 Monza, Milan (IT); Strepparola, Ezio, Dr., I-24047 Treviglio, Bergamo (IT)
(74) Representative: Barz, Peter, Dr.

(56) References cited:
- US-A- 3 007 790
- REPORTS RES. LAB. ASAHI GLASS CO., LTD., VOL. 35, NO. 2, 1985, PAGES 165-174; S. SAMEJIMA ET AL, "A NEW ROUTE TO THE PREPARATION OF BROMODIFLUOROACETYL FLUORIDE"

## Description

The present invention relates to a process for preparing bromodifluoroacetylfluoride of formula CF₂Br-COF.

Bromodifluoroacetylfluoride is a useful intermediate for the preparation of various fluoro-compounds. In particular, it can be converted into omega-bromo-perfluorovinylethers, for example through the following reactions: 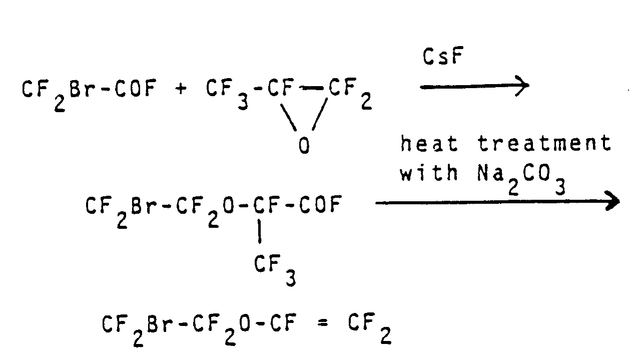
In turn, the omega-bromo-perfluorovinylethers can be used as monomers for the copolymerization with fluorinated olefins. Thereby it is possible to obtain elastomers capable of radical cross-linking.

According to GB-A-2,087,380, CF₂Br-COF may be prepared by treating CF₂ = CFX (wherein X is F, Cl, Br or I) with Br₂ and SO₃ and heating the resulting intermediate product in fuming H₂SO₄ or in KF/sulpholane. This process provides a limited selectivity.

According to C.J. Schack, D. Pilipovich and J.F. Hon, Inorganic chemistry, 12(4), 1973, 897-900, CF₂Br-COF may be prepared by reacting CF₂ = CFX (wherein X is F or Cl) with BrOClO₃ and heating the resulting bromoperfluoroperchlorate with CsF or KF. This process affords only low yields.

Thus, it is an object of the present invention to provide a process for preparing CF₂Br-COF which allows to avoid the drawbacks of the above methods and which provides, by working according to particular conditions, substantially quantitative yields of CF₂Br-COF.

This and still further objects are achieved by the process of the present invention for preparing bromodifluoroacetylfluoride. Said process is characterized in that a gaseous stream of oxygen-diluted ozone is reacted, at temperatures ranging from -100° to +80°C, with 1,4-dibromohexafluorobutene-2 of formula CF₂Br-CF=CF-CF₂Br, optionally dissolved in a solvent which is inert under the reaction conditions, and the ozonide obtained thereby is subjected to a heat treatment, at temperatures ranging from 100° to 300°C, which affords the desired product, bromodifluoroacetylfluoride.

The first reaction step may be represented as follows: 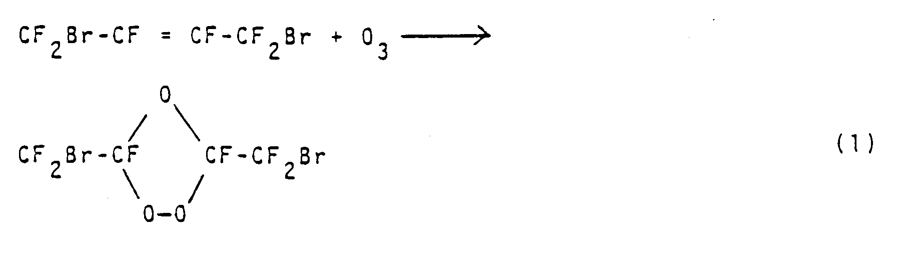
The second reaction step is as follows:

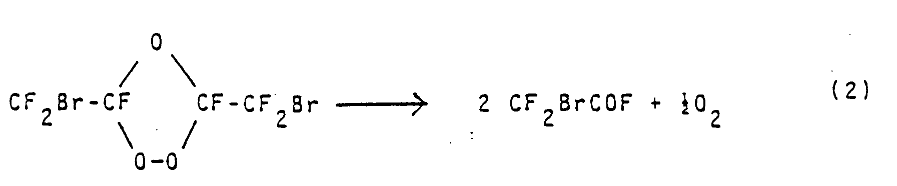

Preferably, in the first step a temperature ranging from -80°to +40°C and in the second step a temperature ranging from 150° to 250°C is employed.

The oxygen/ozone mixture usually contains from 0.01 to 10% by volume of O₃; preferably, it contains from 1 to 6% by volume.

The two reaction steps usually are conducted at atmospheric pressure. However, it also is possible to operate, in the first and/or second step at a pressure higher or lower than atmospheric pressure.

According to a first preferred embodiment of the present invention, the first step is conducted without solvent or with a first class of solvents which is liquid under the temperature and pressure conditions used in the first step and is gaseous under the temperature and pressure conditions used in the second step. Upon completion of the first step, the reaction mixture is transferred to a packed column, wherein the second step takes place. The packing material has to show a high surface area (usually at least 60 m²/g), must be stable under the temperature conditions of the second step and must be inert, under said temperature conditions, towards bromodifluoroacetylfluoride.

Suitable packing materials are, for example, activated carbon, styrene-divinylbenzene resins, zeolites and oxides of elements of Groups IIIA and IVA of the Periodic Table. Preferably activated carbon or styrene-divinylbenzene resins are used.

Generally, the surface area of the packing material does not exceed 1,000 m²/g and materials having a surface area ranging from about 300 to about 500 m²/g are preferred.

Examples of suitable solvents of the above first class are:
i) chlorofluorocarbons, such as CFCl₃, CF₂Cl-CFCl₂ and the isomer mixtures of C₃F₇Cl₃ and C₄F₆Cl₄,
ii) perfluoroalkanes having 6 to 9 carbon atoms,
iii) perfluoropolyethers having a pour point lower than the temperature utilized in the first step, e.g., perfluorodiglyme, perfluorotetraglyme and the perfluoropolyethers Galden® DO1 (produced by Montefluos).

This first embodiment is particularly preferred because it secures practically quantitative yields of CF₂Br-COF. In accordance with a second preferred embodiment of the invention, the first step is conducted without solvent or with a solvent of the above first class or with a second class of solvents which is liquid under the temperature and pressure conditions of both the first step and the second step. The second step is conducted in the absence of packing material with the reaction mixture of the first step or, when a solvent of the first class has been used in the first step, said solvent may be replaced by a solvent of the second class.

More precisely, in accordance with said second embodiment, when a solvent of the first class is used in the first step, it is possible to also use it in the second step (wherein it is transformed to the gaseous state), or it can be replaced by a solvent of the second class before proceeding to the second step.

For replacing the solvent, it is possible to operate, e.g., as follows: the solvent of the second class is added to the solution of the first step, whereafter the solvent of the first class is removed by distillation.

Suitable solvents of the second class are, for example, chlorotrifluoroethylene telomers having a degree of telomerization of from 3 to 6 and perfluoropolyethers having a boiling point higher than the temperature used in the second step and a pour point lower than the temperature employed in the first step. Among the suitable perfluoropolyethers, for example, the following commercial products can be mentioned: Galden®, having a kinematic viscosity at 20°C of more than 2 cSt, Fomblin® Y and Fomblin® Z, all being produced by Montefluos.

When a solvent is used in the first step, the O₃/O₂ stream is introduced into the liquid phase of 1,4-dibromohexafluorobutene-2 and the solvent until achieving, preferably, a complete conversion of the olefin. It is also possible to use a slight O₃/O₂ excess with respect to the stoichiometry of reaction (1), but, for safety reasons, it is advisable that the excess of O₃ does not exceed 5% by moles of the stoichiometric amount with respect to the olefin. It is also possible to stop the O₃/O₂ flow when the olefin conversion is still incomplete. In this case a conversion of at least 10% is generally aimed at.

When no solvent is used in the first step, it is advisable, for safety reasons, to stop the O₃/O₂ flow before the olefin conversion exceeds 50%.

When the second step is carried out in a packed column, it is possible to convey to the column, along with the reaction mixture, also an inert gas stream, for example of nitrogen or helium. Usually, the volume ratio of inert gas stream to ozonide stream ranges from 2:1 to 4:1.

The ozonide decomposition can be carried out in the presence of an aprotic reducing agent.

Examples of suitable aprotic reducing agents are:
a) fluorinated olefins, in particular those containing 2 to 4 carbon atoms;
b) organic sulphides, for example dimethylsulphide;
c) phosphines, for example triphenylphosphine.

The concentration of 1,4-dibromohexafluorobutene-2 in the solvent, if any, can be varied over a wide range. Generally, a concentration ranging form 0.5 to 70% by volume and, preferably, from 5 to 40% is used.

When in the second step the solvent is replaced, the ozonide concentration preferably is maintained unchanged.

When a reducing agent is utilized in the second step, it usually is used in an at least stoichiometric amount with respect to the oxygen atom which is liberated in reaction (2).

Upon completion of the second step, if a solvent of the first class has been used, CF₂Br-COF may be purified by known methods, for example distillation, after condensation of the gaseous mixture. If a solvent of the second class has been used, CF₂Br-COF already condenses practically in the pure state.

The following examples are given for merely illustrative purposes and do not limit the scope of the present invention.

### EXAMPLE 1

25 g (0.078 moles) of CF₂Br-CF = CF-CF₂Br and 50 ml of CFCl₃ were placed into a cylindrical 100 ml reactor of AISI steel, equipped with a stand dipping pipe for the gas feeding and with a vent to the atmosphere for the outlet of said gases. After cooling to -80°C, a gaseous stream of O₂ and O₃ at 4% by volume of O₃ was introduced at a flow rate of 10 Nl/h, for 4.5 hours.

A nitrogen stream was then fed for 30 minutes to remove the unreacted ozone which had remained dissolved in the reaction mixture. The solution was allowed to come to room temperature again and then was dropped, at a rate of 20 g/h, under a nitrogen stream of 4 N l/h, into a steel pipe heated to 150°C and filled with styrene-divinylbenzene resin Amberlite® XAD-2, produced by Rohm and Haas, having a surface area of 330 m²/g. The pipe had an internal diameter of 4 mm and a length of 1 m.

The gaseous products leaving the pipe were condensed at -80°C. Thus, 27 g of CF₂Br-COF (0.15 moles) were obtained in admixture with the solvent, which could be removed by distillation.

### EXAMPLE 2

The procedure of example 1 was followed, except for the following modalities:
- no solvent was used;
- the O₂/O₃ stream was introduced for 2 hours.

The gaseous product condensed upon completion of the second step was composed of 12.6 g of CF₂Br-COF (0.071 moles) and 13.5 g of unreacted CF₂Br-CF = CF - CF₂Br (0.042 moles).

### EXAMPLE 3

The first step was carried out according to the procedure of example 1, except that the O₃/O₂ stream was introduced at a flow rate of 20 N l/h for 4 hours.

A nitrogen stream was then fed for 1 hour in order to remove the unreacted ozone which had remained dissolved in the solution. The solution as allowed to come to room temperature again and then was heated to 150°C for 4 hours. Upon completion of the reaction, the gaseous products leaving the reactor were condensed at -80°C. Thus, about 17 g of CF₂BrCOF (about 0.096 moles), in admixture with the solvent, were obtained.

## Claims

1. Process for preparing bromodifluoroacetylfluoride, wherein a gaseous stream of oxygen-diluted ozone is reacted, at a temperature of from -100° to +80°C, with 1,4-dibromohexafluorobutene-2, optionally dissolved in a solvent inert under the reaction conditions, and the resulting ozonide is subjected to a heat treatment at temperatures of from 100° to 300°C.

2. Process according to claim 1, wherein the reaction between ozone and 1,4-dibromohexafluorobutene-2 is conducted at a temperature of from -80° to +40°C.

3. Process according to any one of claims 1 and 2, wherein the ozonide is treated at a temperature of from 150° to 250°C.

4. Process according to one or more of the preceding claims, wherein the gaseous stream of oxygen-diluted ozone contains from 0.01 to 10% by volume of ozone.

5. Process according to one or more of the preceding claims, wherein the reaction between ozone and 1,4-dibromohexafluorobutene-2 (first step) is conducted without solvent or in the presence of a first class of solvents which is liquid under the temperature and pressure conditions employed in the first step and is gaseous under the temperature and pressure conditions employed in the ozonide heat treatment (second step) and wherein, at the end of the first step, the reaction mixture is transferred to a column packed with a material of high surface area which is stable under the temperature conditions of the second step and is inert, under said temperature conditions, towards bromodifluoroacetylfluoride.

6. Process according to claim 5, wherein the packing material is selected from activated carbon, styrene-divinylbenzene resins, zeolites and oxides of elements of Groups IIIA and IVA of the Periodic Table.

7. Process according to one or more of claims 1 to 4, wherein the first step is conducted without solvent or with the first class of solvents as defined in claim 5 or with a second class of solvents which is liquid under the temperature and pressure conditions of both the first and the second step and wherein the second step is conducted, in the absence of packing material, with the reaction mixture of the first step, or, in case a solvent of the first class has been used in the first step, under substitution of said solvent by a solvent of the second class.

8. Process according to one or more of claims 5 to 7, wherein the solvent of the first class is selected from chlorofluorocarbons, perfluoroalkanes having 6 to 9 carbon atoms, perfluoropolyethers having a pour point lower than the temperature used in the first step and mixtures thereof.

9. Process according to one or more of claims 7 and 8, wherein the solvent of the second class is selected from chlorotrifluoroethylene telomers having a degree of telomerization of from 3 to 6, perfluoropolyethers having a boiling point higher than the temperature used in the second step and a pour point lower than the temperature employed in the first step and mixtures thereof.

## Patentansprüche

1. Verfahren zur Herstellung von Bromdifluoracetylfluorid, bei welchem ein gasförmiger Strom von mit Sauerstoff verdünntem Ozon bei einer Temperatur von -100° bis +80°C mit 1,4-Dibromhexafluorbuten-2, gegebenenfalls in einem unter den Reaktionsbedingungen inerten Lösungsmittel gelöst, umgesetzt wird und das resultierende Ozonid bei Temperaturen von 100° bis 300°C einer Wärmebehandlung unterzogen wird.

2. Verfahren nach Anspruch 1, bei welchem die Reaktion zwischen Ozon und 1,4-Dibromhexafluorbuten-2 bei einer Temperatur von -80° bis +40°C durchgeführt wird.

3. Verfahren nach irgendeinem der Ansprüche 1 und 2, bei welchem das Ozonid bei einer Temperatur von 150° bis 250°C behandelt wird.

4. Verfahren nach einem oder mehreren der vorangehenden Ansprüche, bei welchem der gasförmige Strom von mit Sauerstoff verdünntem Ozon 0,01 bis 10 Volumen-% Ozon enthält.

5. Verfahren nach einem oder mehreren der vorangehenden Ansprüche, bei welchem die Reaktion zwischen Ozon und 1,4-Dibromhexafluorbuten-2 (erste Stufe) ohne Lösungsmittel oder in Anwesenheit einer ersten Klasse von Lösungsmitteln, die unter den in der ersten Stufe eingesetzten Temperatur- und Druckbedingungen flüssig ist und unter den Temperatur- und Druckbedingungen, die in der Ozonid-Wärmebehandlung (zweite Stufe) eingesetzt werden, gasförmig ist, durchgeführt wird, und bei welchem am Ende der ersten Stufe die Reaktionsmischung in eine mit einem Material mit hoher Oberfläche, das unter den Temperaturbedingungen der zweiten Stufe stabil und unter diesen Temperaturbedingungen gegenüber Bromdifluoracetylfluorid inert ist, gepackte Säule überführt wird.

6. Verfahren nach Anspruch 5, bei welchem das Packungsmaterial aus aktiviertem Kohlenstoff, Styrol-Divinylbenzol-Harzen, Zeolithen und Oxiden von Elementen der Gruppen IIIA und IVA des Periodensystems ausgewählt wird.

7. Verfahren nach einem oder mehreren der Ansprüche 1 bis 4, bei welchem die erste Stufe ohne Lösungsmittel oder mit der ersten Klasse von Lösungsmitteln, wie sie in Anspruch 5 definiert ist, oder mit einer zweiten Klasse von Lösungsmitteln, die unter den Temperatur- und Druckbedingungen sowohl der ersten als auch der zweiten Stufe flüssig ist, durchgeführt wird, und bei welchem die zweite Stufe in Abwesenheit von Packungsmaterial mit der Reaktionsmischung der ersten Stufe oder im Falle der Verwendung eines Lösungsmittels der ersten Klasse in der ersten Stufe unter Ersatz des Lösungsmittels durch ein Lösungsmittel der zweiten Klasse durchgeführt wird.

8. Verfahren nach einem oder mehreren der Ansprüche 5 bis 7, bei welchem das Lösungsmittel der ersten Klasse aus Chlorfluorkohlenstoffen, Perfluoralkanen mit 6 bis 9 Kohlenstoffatomen, Perfluorpolyethern mit einem Stockpunkt unter der Temperatur, die in der ersten Stufe verwendet wird, und Mischungen davon ausgewählt wird.

9. Verfahren nach einem oder mehreren der Ansprüche 7 und 8, bei welchem das Lösungsmittel der zweiten Klasse aus Chlortrifluorethylen-Telomeren mit einem Telomerisationsgrad von 3 bis 6, Perfluorpolyethern mit einem Siedepunkt, der höher ist als die Temperatur, die in der Zweiten Stufe eingesetzt wird, und einem Stockpunkt, der niedriger ist als die Temperatur, die in der ersten Stufe verwendet wird, und Mischungen davon ausgewählt wird.

## Revendications

1. Procédé de préparation du fluorure de bromodifluoroacétyle, suivant lequel un courant gazeux d'ozone dilué par de l'oxygène est mis à réagir, à une température de -100° à +80°C, avec du 1,4-dibromohexafluorobutène-2, facultativement dissous dans un solvant inerte dans les conditions de la réaction, et l'ozonide résultant est soumis à un traitement thermique à des températures de 100° à 300°C.

2. Procédé selon la revendication 1, dans lequel la réaction entre l'ozone et le 1,4-dibromohexafluorobutène-2 est conduite à une température de -80° à +40°C.

3. Procédé selon l'une des revendications 1 et 2, dans lequel l'ozonide est traité à une température de 150° à 250°C.

4. Procédé selon une ou plusieurs des revendications précédentes, dans lequel le courant gazeux d'ozone dilué par de l'oxygène contient de 0,01 à 10% en volume d'ozone.

5. Procédé selon une ou plusieurs des revendications précédentes, dans lequel la réaction entre l'ozone et le 1,4-dibromohexafluorobutène-2 (première étape) est conduite sans solvant ou en présence d'une première classe de solvants qui sont liquides dans les conditions de température et de pression employées à la première étape, et sont gazeux dans les conditions de température et de pression employées dans le traitement thermique de l'ozonide (seconde étape), et dans lequel, à la fin de la première étape, le mélange réactionnel est transféré dans une colonne garnie d'une matière de surface spécifique élevée, qui est stable dans les conditions de température de la seconde étape, et est inerte, dans lesdites conditions de température, envers le fluorure de bromodifluoroacétyle.

6. Procédé selon la revendication 5, dans lequel la matière de garnissage est choisie parmi le charbon actif, les résines styrène-divinylbenzène, les zéolithes et les oxydes d'éléments des Groupes IIIA et IVA de la Classification Périodique.

7. Procédé selon une ou plusieurs des revendications 1 à 4, dans lequel la première étape est conduite sans solvant ou avec la première classe de solvants telle que définie à la revendication 5 ou avec une seconde classe de solvants qui sont liquides dans les conditions de température et de pression à la fois de la première et de la seconde étape, et dans lequel la seconde étape est conduite, en l'absence de matière de garnissage, avec le mélange réactionnel de la première étape, ou, dans le cas où un solvant de la première classe a été utilisé à la première étape, avec remplacement dudit solvant par un solvant de la seconde classe.

8. Procédé selon une ou plusieurs des revendications 5 à 7, dans lequel le solvant de la première classe est choisi parmi les chlorofluorocarbures, les perfluoroalcanes ayant 6 à 9 atomes de carbone, les perfluoropolyéthers ayant un point d'écoulement inférieur à la température utilisée à la première étape, et leurs mélanges.

9. Procédé selon une ou plusieurs des revendications 7 et 8, dans lequel le solvant de la seconde classe est choisi parmi les télomères de chlorotrifluoroéthylène ayant un degré de télomérisation de 3 à 6, les perfluoropolyéthers ayant un point d'ébullition supérieur à la température utilisée à la seconde étape, et un point d'écoulement inférieur à la température employée à la première étape, et leurs mélanges.
